(19) Europäisches Patentamt
European Patent Office
Office européen des brevets

(11) Publication number: **0 196 824 A2**

(12) # EUROPEAN PATENT APPLICATION

(21) Application number: **86302020.2**

(22) Date of filing: **19.03.86**

(51) Int. Cl.[4]: **A 61 K 7/06**

(30) Priority: **21.03.85 GB 8507349**

(43) Date of publication of application: **08.10.86 Bulletin 86/41**

(84) Designated Contracting States: **AT BE CH DE FR GB IT LI NL**

(71) Applicant: **THE PROCTER & GAMBLE COMPANY**
**One Procter & Gamble Plaza**
**Cincinnati Ohio 45202(US)**

(84) Designated Contracting States:
**BE CH DE FR IT LI NL AT**

(71) Applicant: **Procter & Gamble Limited**
**Hedley House**
**Gosforth Newcastle upon Tyne NE99 1EE(GB)**

(84) Designated Contracting States:
**GB**

(72) Inventor: **Bell, Terence Campbell**
**9 Julian Street**
**South Shields Newcastle upon Tyne(GB)**

(74) Representative: **Brooks, Maxim Courtney et al,**
**Procter & Gamble (NTC) Limited Whitley Road**
**Longbenton**
**Newcastle-upon-Tyne NE12 9TS(GB)**

(54) **Hair care compositions.**

(57) Anti-microbial compositions for use in shampoos, rinses and the like for treatment of dandruff, containing a mixture of a water-insoluble salt of 1-hydroxy-2-pyridinethione (I) and an imidazolyl ketone derivative of the general formula II.

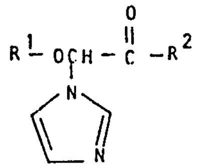

(II)

wherein $R^1$ and $R^2$ are independently selected from optionally substituted alkyl, alkenyl, alkynyl, cycloalkyl, aryl and alkanyl groups.

The compositions display improved anti-dandruff efficacy.

# HAIR CARE COMPOSITIONS

The present invention relates to antimicrobial compositions and to their use in hair care. In particular it relates to hair care compositions having improved antidandruff efficacy.

Dandruff is a widespread problem, some 50%-60% of the overall population suffering with dandruff at varying periods of their lives and to varying degrees. Although the cause or causes of dandruff are still not precisely settled, the microorganism Pityrosporum Ovale is strongly implicated in the etiology of the scurf-formation process.

The use of pyridinethione salts as antidandruff agents in shampoos and hair rinse is well known. US-A-3,236,733 discloses detergent compositions containing such salts. Other references include US-A-2,809,971 and US-A-3,723,325.

It is also known that certain imidazolyl ketone derivatives are effective as antimicrobial agents with specific application to dandruff control. US-A-4,329,334, US-A-4,329,335 and US-A-4,329,336 are relevant in this respect.

Both the pyridinethione salts and the imidazolyl ketones have been clinically proven to reduce the level of dandruff when used regularly over, for example, a four week period. Typically, there is a reduction in mean dandruff grades of about 65%-70% after four weeks twice weekly usage. Continued treatment can provide some further marginal improvements but on average, these materials reach a plateau in anti-dandruff effectiveness. Improvements in the level of dandruff reduction or in the rate at which dandruff reduction is achieved, would therefore be highly desirable.

The present invention is based on the finding that a combination of certain pyridinethiones and imidazolyl ketones provide improved antidandruff efficacy when used in hair compositions such as shampoos, cream rinses and the like.

Accordingly, the present invention provides an antimicrobial composition comprising a mixture of

(a) a water-insoluble salt of 1-hydroxy-2-pyridinethione (I), and

(b) an imidazolyl ketone derivative of the general formula II

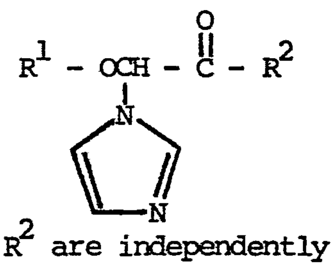

(II)

wherein $R^1$ and $R^2$ are independently selected from optionally substituted alkyl, alkenyl, alkynyl, cycloalkyl, aryl and alkaryl groups.

The compositions of the invention comprise two essential components, a pyridinethione component (I) and an imidazolyl ketone component (II).

The pyridinethione component of the present invention can generally be defined as water-insoluble salts of 1-hydroxy-2-pyridinethione which has the following structural formula in tautomeric form, the sulfur being attached to the No. 2 position in the pyridine ring.

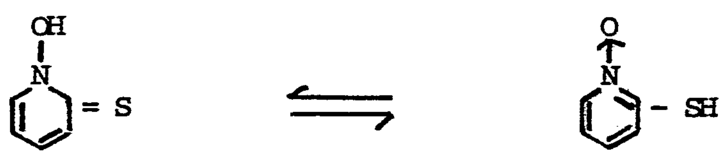

The salts result from substitution of the hydrogen of one of the tautomeric forms by the appropriate salt cation. Depending, of course, on the valence of the salt cation involved there may be more than one of the pyridinethione rings in the compound.

Preferred salts are formed from heavy metals such as zinc, tin, cadmium, magnesium, aluminium and zirconium. The preferred heavy metal is zinc. Other cations such as sodium are also suitable, however.

The pyridinethiones generally take the form of water-insoluble particles. These can have an individual particle size of at least 1 micrometer. Preferred materials, however, are pyridinethione crystals in the form of predominantly flat platelets which have a mean sphericity of less than about 0.65, preferably from about 0.20 to about 0.54 and a median particle size of at least about 2 micrometres, the particle size being expressed as the median equivalent diameter of a sphere of equal volume. The upper limit on mean particle size is not critical but generally can range up to about 25 micrometers, measured on the same basis. The median diameters are on a mass basis with 50% of the mass of particles falling on either side of the value given.

The diameter of a sphere of equivalent volume for a particle can be determined by a variety of sedimentation techniques which are based on Stokes' Law for the settling velocity of a particle in a fluid. Such techniques are described in Stockham, J.D. and Fochtman, E.G., Particle Size Analysis, Ann Arbor Science, 1978. An approach for determining the median equivalent spherical diameter based on volume, $\bar{d}_v$, is shown in EP-A-34, 385, Example II.

The sphericity of a particle is also described by Stockham and Fochtman at page 113 as $(d_v/d_s)^2$, where $d_v$ is the diameter of a sphere of equivalent volume, supra, and $d_s$ is the diameter of a sphere of equivalent area. As used herein, however, the mean sphericity is $(\bar{d}_V/\bar{d}_S)^2$, or the surface area of spheres having equivalent volume distribution divided by the actual surface area of particles as measured. A technique for determining actual surface area is shown in the examples using the BET technique described by Stockham and Fochtman at page 122.

In preferred embodiments, pyridonethione antidandruff agents herein are at least partly in agglomerated form, the agglomerate size preferably being such that at least about 20% by weight, more preferably at least about 50% by weight of the pyridinethione is in the form of agglomerates having an equivalent spherical diameter of at least 5, preferably up to 30, more preferably 10 to 20 microns.

The imidazolyl ketone component (II) of the present compositions has the general formula

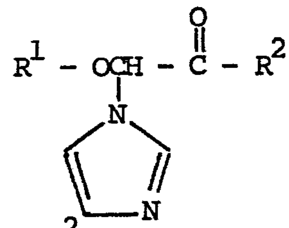

(II)

Preferably $R^1$ and $R^2$ are independently selected from optionally substituted $C_1$-$C_8$ alkyl, alkenyl and alkynyl; optionally substituted aryl and $C_{1-4}$ alkaryl; and optionally substituted $C_5$-$C_7$ cycloalkyl and cycloalkenyl groups. Of the above, $R^1$ is preferably an optionally halo substituted (e.g. chloro or bromo or fluoro) aryl or $C_1$-$C_4$ alkaryl group and $R^2$ is preferably a $C_1$-$C_8$ straight chain or branched alkyl or alkenyl group. The highly preferred imidazolyl ketone is 1-(p-chlorophenoxy)-1-(1H-imidazol-1-yl)-3,3-dimethyl-2-butanone.

The pyridinethione/imidazolyl ketone mixtures specified herein are useful in a variety of hair care compositions as antidandruff aids. The total level of pyridinethione and imidazolyl ketone is generally from about 0.1% to about 5%, preferably from about 0.5% to about 3% by weight of composition. The weight ratio of pyridinethione salt to imidazolyl ketone is generally from about 10:1 to about 1:10, preferably from about 5:1 to about 1:5. Individually, the pyridinethione salts and imidazolyl ketones are each generally present at a level of from about 0.05% to about 3%, preferably from about 0.2% to about 2% by weight of composition.

Included among the hair care compositions are shampoos, creme rinses, hair tonics, hair conditioners, hair sprays, lotions and creams and many others. Shampoos are the preferred compositions and components generally found in such compositions are given below.

One essential component of the antidandruff shampoos herein is a surfactant. The term "surfactant" as used herein is intended to denote soap and nonsoap surfactants. The surfactant component usually comprises from about 5% to about 50% by weight of the composition, preferably from about 10% to about 20%.

Any nonsoap surfactant is suitable for use including anionic, nonionic, amphoteric, zwitterionic and cationic types.

Examples of suitable soaps are the sodium, potassium, ammonium and alkanol ammonium salts of higher fatty acids (those having 10-20 carbon atoms). Anionic nonsoap surfactants can be exemplified by the alkali metal salts of organic sulfuric reaction products having in their molecular structure an alkyl radical containing from 8 - 22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols ($C_8$ - $C_{18}$ carbon atoms), sodium coconut oil fatty acid monoglyceride sulfates and sulfonates; sodium or potassium salts of sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and others known in the art.

Nonionic surfactants can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from about 6 to 12 carbon atoms in either a straight chain or branched chain configuration, with ethylene oxide, the said ethylene oxide being present in amounts equal to 10 to 60 moles of ethylene oxide per mole of alkyl phenol. The alkyl substituent in such compounds may be derived from polymerixed propylene, diisobutylene, octane, or nonane, for example.

2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from about 40% to about 80% polyoxyethylene by weight and having a molecular weight of from about 5,000 to about 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.

3. The condensation product of aliphatic alcohols having from 8 to 18 carbon atoms, in either straight chain or branched chain configuration, with ethylene oxide, e.g., a coconut alcohol ethylene oxide condensate having from 10 to 30 moles of ethylene oxide per mole of coconut alcohol, the coconut alcohol fraction having from 10 to 14 carbon atoms.

 

4. Long chain tertiary amine oxides corresponding to the following general formula:

$$R_1R_2R_3N \rightarrow O$$

wherein $R_1$ contains an alkyl, alkenyl or monohydroxy alkyl radical of from about 8 to about 18 carbon atoms, from 0 to about 10 ethylene oxide moieties, and from 0 to 1 glyceryl moiety, and $R_2$ and $R_3$ contain from 1 to about 3 carbon atoms and from 0 to about 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyldi(2-hydroxyethyl)amine oxide, dimethyloctylamine oxide, dimethyldecylamine oxide, dimethyltetradecylamine oxide, 3,6,9-trioxaheptadecyldiethylamine oxide, di(2-hydroxyethyl)-tetradecylamine oxide, 2-dodecoxyethyldimethylamine oxide, 3-dodeoxycy-2-hydroxypropyldi(3-hydroxypropyl)amine oxide, dimethylhexadecylamine oxide.

Zwitterionic surfactants can be exemplified by those which can be broadly described as derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate, or phosphonate. A general formula for these compounds is:

$$R^2 - \overset{\overset{\displaystyle (R^3)_x}{|}}{Y^{(+)}} - CH_2 - R^4 - Z^{(-)}$$

wherein $R^2$ contains an alkyl, alkenyl, or hydroxy alkyl radical of from about 8 to about 18 carbon atoms, from 0 to about 10 ethylene oxide moieties and from 0 to 1 glyceryl moiety; Y is selected from nitrogen, phosphorus, and sulfur atoms, $R^3$ is an alkyl or monohydroxyalkyl group containing 1 to about 3 carbon atoms; x is 1 when Y is a sulfur atom and 2 when Y is a nitrogen or phosphorus atom; $R^4$ is an alkylene or hydroxyalkylene of from 1 to about 4 carbon atoms and Z is a radical selected from carboxylate, sulfonate, sulfate, phosphonate, and phosphate groups.

Examples of amphoteric surfactants which can be used in the compositions of the present invention can be broadly described as derivatives of aliphatic secondary and tertiary amines in which the aliphatic radical can be straight chain or branched and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecylaminopropionate, sodium 3-dodecylaminopropane sulfonate, N-alkyltaurines such as the one prepared by reacting dodecylamine with sodium isethionate according to the teaching of US-A-2,658,072, N-higher alkyl aspartic acids such as those produced according to the teaching of US-A-2,438,091, and the products sold under the trade name "Miranol" and described in US-A-2,528,378.

Many cationic surfactants are known to the art. By way of example, the following may be mentioned:

dodecyltrimethylammonium chloride;
nonylbenzylethyldimethylammonium nitrate;
tetradecylpyridinium bromide;
laurylpyridinium chloride;

cetylpyridinium chloride;
laurylpyridinium chloride;
laurylisoquinolium bromide;
ditallow(hydrogenated)dimethyl ammonium chloride
dilauryldimethylammonium chloride; and
stearalkonium chloride.

Many additional nonsoap surfactants are described in McCUTCHEON'S, DETERGENTS AND EMULSIFIERS, 1979 ANNUAL, published by Allured Publishing Corporation.

The above-mentioned surfactants can be used alone or in combination in the shampoo compositions of the present invention.

The compositions of the invention will generally contain a suspending agent. The suspending agent is preferably substantially crystalline in form. Also, preferred suspending agents are non-polymeric and non-clay in nature, such materials being found to have a deleterious effect on overall anti-dandruff efficacy. Accordingly, the compositions herein are preferably substantially free of nonionic and anionic polymeric and clay-type suspending agents. By "substantially free" it is meant not more than about 20 ppm of these materials. Preferred suspending agents include ethylene glycol and glycerol esters of fatty acids having from about 16 to about 22 carbon atoms. Both ethylene glycol mono and distearate are examples of these esters.

Other useful suspending agents are alkanolamides of fatty acids having from about 16 to about 22 carbon atoms, preferably about 16 to 18 carbon atoms. Suitable examples of these agents include stearic monoethanolamide, stearic diethanolamide, stearic monoisopropanolamide, stearic diethanolamide distearate and mixtures thereof.

Still other suitable suspending agents are $C_{16}$-$C_{22}$ alkyl dimethylamine oxides such as stearyl dimethyl amine oxide and $C_{16}$-$C_{22}$ fatty acid amidoalkylamines such as stearamidoethyldiethylamine. Mixtures of suspending agents are also acceptable, for example, a mixture of glyceryl stearate and stearamidoethyldiethylamine. When present, these suspending agents are at levels of from about 1% to about 7%, preferably from about 2% to about 6%. These agents may also serve to give pearlescence to the product.

The antidandruff shampoos herein can contain a variety of non-essential optional ingredients suitable for rendering such compositions more stable and desirable. Such conventional optional ingredients are well known to those skilled in the art, e.g., pearlescent materials such as bismuth oxychloride, guanine and titanium dioxide coated mica, preservatives such as benzyl alcohol, methyl paraben, propyl paraben and imidazolidinyl urea; thickeners and viscosity modifiers such as sodium chloride and sodium sulfate; and sequestering agents such as disodium ethylenediamine tetraacetate.

An amide is a preferred optional component present in the shampoo aspect of the present invention. Any of the alkanolamides of fatty acids useful in shampoo compositions can be used. Generally, these include mono and diethanolamides of fatty acids having from about 8 to about 14 carbon atoms. Preferred compounds include coconut monoethanolamide, lauric or coconut diethanolamide, and mixtures thereof. The amide is present at a level of from about 2.0% to about 4.0%. It is believed that a more richer lathering, more stable product results when amides of these types are combined with the other components of the invention at hand.

Minor ingredients such as perfumes, dyes and coloring agents can also be added to the instant compositions to improve their consumer acceptability. If present, such agents generally comprise from about 0.1% to 2.0% by weight of the composition.

The shampoos herein are preferably in the form of liquids or creams in which water is the principal diluent. The level of water in the compositions is typically from about 35% to about 90% by weight.

Creme rinses, hair tonics and other hair care compositions as well as shampoos can contain the pyridinethione/imidazolyl ketone mixtures of the present invention. These compositions can contain a variety of other components including some of those described above for

shampoos. Creme rinses generally contain a cationic surfactant similar to those described above, particularly stearalkonium chloride and ditallow dimethyl ammonium chloride at a level of from about 0.1% to about 5%, preferably from about 0.5% to about 2%. Cream rinses can also contain a fatty alcohol such as cetyl alcohol, stearyl alcohol and mixtures thereof at a level of from about 0.5% to about 5%, preferably from about 1.0% to about 3.0% by weight of composition.

The hair care compositions herein have a pH generally in the range from about 3 to about 8. Preferred compositions are acidic however, highly preferred compositions having a pH in the range from about 3.5 to about 6.5, especially from about 4 to about 6. The pH is measured on a 1% solution basis. A preferred pH regulating agent is a ctric acid/sodium citrate buffer. Other acidulating agents such as phosphoric acid, succinic acid etc. can also be used, however.

A process for preparing the compositions herein comprises preparing a surfactant premix comprising from about 50% to about 90% by weight of final composition of water and from about 2% to about 30% by weight of final composition of surfactant at a temperature in the range from about 71°C to 88°C, cooling the premix to a temperature in the range from about 21°C to about 49°C, preferably from about 21°C to about 46°C, and thereafter adding the pyridinethione and imidazolyl ketone antidandruff agents to the cooled premix. Suspending agent, when present, is added to the surfactant premix at the high temperature stage and the premix is maintained after cooling for a sufficient period to achieve substantial crystallization of the suspending agent prior to adding the pyridinethione and imidazolyl ketone antidandruff agents. Alternatively, the imidazolyl ketone can be added to the surfactant premix at the high temperature stage. The

step of adding the pyridinethione is preferably performed under low shear (non-turbulent flow) conditions.

The following Examples further describe and demonstrate the preferred embodiments within the scope of the present invention. Unless otherwise indicated, all percentages herein are by weight.

In the Examples which follow, the abbreviations have the following designations.

| | |
|---|---|
| ZPT1 | Zinc Pyridinethione<br>Median Equivalent Spherical Diameter = 5.4 microns<br>Mean Sphericity = 0.25 |
| ZPT2 | Zinc Pyridinethione<br>Median Equivalent Spherical Diameter = 1.25 microns<br>Mean Sphericity = 0.75 |
| ZPT3 | Agglomerated Zinc Pyridinethione - 20% having an equivalent spherical diameter of at least 5 microns |
| ZPT4 | Agglomerated Zinc Pyridinethione platelets - 50% having an equivalent spherical diameter of at least 13 microns |
| CPIB | 1-(p-chlorophenoxy)-1-(1H-imidazol-1-yl)-3,3-dimethyl-2-butanone |
| $NH_4AS$ | Ammonium $C_{12}$ alkyl sulfate (29% aq. soln.) |
| TEA AS | Triethanolamine $C_{12}$ alkyl sulfate (35% aq. soln.) |
| $NH_4$ $AE_2S$ | Ammonium $C_{12}$ alkyl $(EO)_2$ sulfate (24% active) |
| AGS | Sodium alkyl glyceryl sulfonate (60%) |
| MEA | Coconut Monoethanoamide |

DEA - Lauric Diethanolamide

LS    Sodium N-lauroyl sarcosinate

CSA   N-Cocoyl Sarcosine Acid

EGDS  Ethylene glycol distearate

Examples I to V

The following are shampoo compositions according to the invention.

| | I | II | III | IV | V | VI | VII |
|---|---|---|---|---|---|---|---|
| ZPT1 | 1 | 0.5 | 0.8 | 0.5 | - | - | - |
| ZPT2 | - | - | - | - | 0.5 | - | - |
| ZPT3 | - | - | - | - | - | 0.5 | - |
| ZPT4 | - | - | - | - | - | - | 0.5 |
| CPIB | 1 | 0.5 | 0.5 | 1 | 1 | 0.5 | 0.5 |
| $NH_4AS$ | 55 | - | - | - | 45 | 28 | 25 |
| TEA AS | - | 50 | - | 55 | - | - | - |
| $NH_4$ $AE_2S$ | - | 3 | - | - | 2 | 40 | 35 |
| AGS | - | - | 60 | - | - | - | - |
| MEA | 3 | 2 | - | 3 | 4 | 3 | 2 |
| DEA | - | - | 2 | - | - | - | - |
| LS | - | - | 12 | - | - | - | - |
| CSA | - | - | 1 | - | - | - | - |
| EGDS | 5 | 4 | 3 | 5 | 3 | 5 | 5 |
| Sodium Chloride | - | - | 5 | - | - | - | - |
| Sodium Citrate | 0.5 | 0.5 | 0.6 | 0.8 | 0.7 | 0.8 | 0.5 |
| Citric Acid | 0.2 | 0.2 | 0.3 | 0.6 | 0.4 | 0.5 | 0.2 |
| Colour solution | 0.1 | 0.1 | 0.12 | 0.2 | 0.1 | 0.1 | 0.1 |
| Perfume | 0.5 | 0.3 | 0.5 | 0.6 | 0.5 | 0.5 | 0.5 |
| Water | To 100% | | | | | | |

The compositions display improved anti-dandruff efficacy.

Examples VIII to X

The following are rinse conditioning compositions according to the invention.

| | VIII | IX | X |
|---|---|---|---|
| ZPT1 | 0.5 | - | - |
| ZPT3 | - | 0.5 | - |
| ZPT4 | - | - | 0.5 |
| CPIB | 0.5 | 0.5 | 0.5 |
| Stearalkonium chloride | 1.6 | 1.2 | 1.8 |
| Cetyl trimethyl ammonium chloride | 1.6 | 1.8 | 1.2 |
| Cetyl alcohol | 1.3 | 1.1 | 1.5 |
| Stearyl alcohol | 1.3 | 1.5 | 1.1 |
| Ceteth-2 | 0.8 | 0.9 | 0.7 |
| Crotein Q (1) | 0.5 | 0.4 | 0.6 |
| Citric acid | 0.1 | 0.2 | 0.1 |
| Sodium chloride | 0.1 | 0.1 | 0.1 |
| Perfume | 0.2 | 0.2 | 0.2 |
| Kathon CG (2) | 0.03 | 0.03 | 0.03 |
| Water | ———To 100%——— | | |

1. A quaternised protein offered by Croda Inc.
2. Preservative offered by Rohm & Haas.

CLAIMS

1. An antimicrobial composition comprising a mixture of
   (a) a water-insoluble salt of 1-hydroxy-2-pyridinethione (I), and
   (b) an imidazolyl ketone derivative of the general formula II

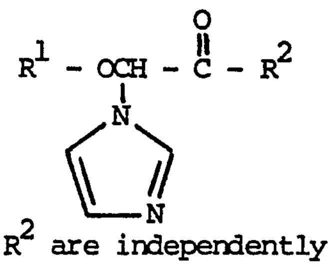 (II)

wherein $R^1$ and $R^2$ are independently selected from optionally substituted alkyl, alkenyl, alkynyl, cycloalkyl, aryl and alkaryl groups.

2. A composition according to Claim 1 wherein the pyridinethione (I) is selected from zinc, tin, magnesium, aluminium, cadmium , zirconium and sodium salts of 1-hydroxy-2-pyridinethione.

3. A composition according to Claim 1 or 2 wherein the pyridinethione (I) is the zinc salt of 1-hydroxy-2-pyridinethione.

4. A composition according to any of Claims 1 to 3 wherein the pyridinethione (I) has a particle size, defined as the mass median equivalent spherical diameter, of at least 1 micrometre, preferably from 2 to 15 micrometres.

5. A composition according to any of Claims 1 to 4 wherein $R^1$ is an optionally halo-substituted aryl or $C_1$-$C_4$ alkaryl group and $R^2$ is a straight chain or branched alkyl or alkaryl group having up to 8 carbon atoms.

6. A composition according to Claim 5 wherein the imidazolyl ketone is 1-(p-chlorophenoxy)-1-(1H-imidazol-1-yl)-3,3-dimethyl -2-butanone.

7. A composition according to any of Claims 1 to 6 wherein pyridinethione (I) and imidazolyl ketone (II) are in weight ratio of from 10:1 to 1:10, preferably from 5:1 to 1:5.

8. A composition according to any of Claims 1 to 7 in the form of an antidandruff hair care composition.

9. An antidandruff hair care composition comprising from 0.1% to 5% by weight of a mixture of

(a) a water-insoluble salt of 1-hydroxy-2-pyridinethione (I), and

(b) an imidazolyl ketone derivative of the general formula II

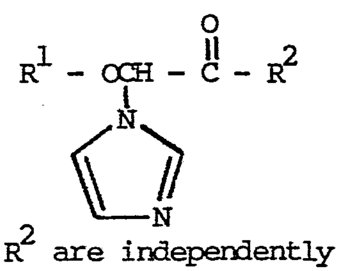

(II) .

wherein $R^1$ and $R^2$ are independently selected from optionally substituted alkyl, alkenyl, alkynyl, cycloalkyl, aryl and alkaryl groups.

10. An antidandruff hair care composition comprising from 0.1% to 5% by weight of a mixture of

(a) a water-insoluble salt of 1-hydroxy-2-pyridinethione (I), and

(b) 1-(p-chlorophenoxy)-1-(1H-imidazol-1-yl)-3,3-dimethyl-2-butanone.

11. A composition according to any of Claims 1 to 10 in the form of a shampoo and which additionally contains from 5% to 50% of surfactant.

12. A composition according to Claim 11 wherein the surfactant comprises an anionic surfactant selected from alkyl sulfates, ethoxylated alkyl sulfates, alkyl glyceryl ether sulfonates and mixtures thereof.

13. A composition according to any of Claims 1 to 12 in the form of a cream rinse and which additionally contains from 0.1% to 5% of a cationic surfactant.